# EUROPEAN PATENT APPLICATION

(11) **EP 3 831 920 A1**
(43) Date of publication of application: **09.06.2021**
(21) Application number: 19306562.0
(22) Date of filing: 03.12.2019
(51) Int. Cl.: C12M 1/107, C12M 1/12, C12M 1/00, C12M 1/34

(54) **INSTALLATION AND METHOD FOR CONTROLLING NH3 CONTENT IN AN ANAEROBIC MEDIUM**

(71) Applicant: SUEZ Groupe, 92040 Paris la Défense Cedex (FR)
(72) Inventor: HADDAD, Mathieu, 92310 SEVRES (FR); PARDO, Pierre-Emmanuel, 91400 ORSAY (FR)
(74) Representative: Marks & Clerk France

(57) **Abstract**

The invention relates to the field of waste treatment and concerns a method for controlling NH3 content in an anaerobic medium of an anaerobic process, characterized in that it comprises:
• a step (100) of providing organic material,
• a step (101) of subjecting said organic material to an anaerobic treatment, thereby producing a digestate and optionally biogas,
• a step (102) of solubilizing CO₂ from a CO₂-containing gas into a liquid medium comprising at least one microorganism population, thereby providing a CO₂-enriched liquid medium,
• a step (104) of subjecting at least part of the CO₂-enriched liquid medium to the anaerobic treatment.

## Description

### TECHNICAL FIELD OF THE INVENTION

The invention relates to the field of waste and sludge treatment and concerns an installation for controlling NH₃ content in an anaerobic medium of an anaerobic tank. The invention further relates to a method for controlling NH₃ content in an anaerobic medium of an anaerobic process, and aims at maintaining NH₃ concentration in the anaerobic tank under a toxicity threshold for the micro-organisms population(s).

### BACKGROUND

Sludge treatment methods usually involve recovery of the carbon contained into the sludge by producing methane-containing gases (biogas), usually through anaerobic digestion. Several problems may impact biogas production, one of which is ammonia poisoning.

Ammonia is indeed known to be toxic to methanogenic microorganisms (*i.e.* microorganisms producing methane upon anaerobic digestion also called methanogens, responsible for biogas production), and thus impacts digestion performance. High ammonia concentrations lead in particular to reduced performances of acetoclastic methanogens (*i.e.* acetate consuming methanogens, in particular Methanosaetaceae), and leads to a microbial population shift towards hydrogen-utilizing methanogens (in particular Methanosarcinaceae, Methanomicrobiales and Methanobacteriales).

Toxic NH₃ levels may occur when the digester is fed with a substrate already highly concentrated in nitrogen, such as municipal waste or thermally pretreated substrate, in particular substrate treated using thermal conditioning, which is also known to favour ammonium release into the liquid phase of the anaerobic medium as compared to conventional digestion, wherein the carbonaceous material to be treated is not subjected to a thermal conditioning.

Normal biological degradation of organic material may also lead to nitrogen enrichment of the digester medium. In anaerobic digestion, protein degradation is indeed the main pathway for nitrogen release, usually as its soluble form: ammonium (NH₄⁺).

Ammonium (NH₄⁺) in the liquid aqueous fraction of the digester medium is then in equilibrium with its corresponding basic form, aqueous ammonia (NH₃), following known chemical equilibrium (1):

NH₄⁺ + H₂O <--> NH₃ + H₃O⁺ (1)

The relative concentrations of NH₃ and NH₄⁺ thus depend on pH and temperature, with higher pH values and temperatures favouring the formation of ammonia, following Lavoisier's Law.

NH₃ toxicity level depends on the methanogens predominant populations. Indeed, methanogens may be classified into two categories: NH₃-tolerant (hydrogen-utilizing methanogens) and NH₃-sensitive micro-organisms (acetoclastic methanogens). The NH₃ toxicity level is about 80 mg/L (*i.e.* 80 mg of NH₃ per liter) for acetoclastic methanogens (such as Methanosaetaceae), and about 750 mg/L for mesophilic hydrogen-utilizing methanogens (such as Methanosarcinaceae, Methanomicrobiales, Methanobacteriales).

In case the NH₃ content in the anaerobic tank varies around or in between these toxicity levels, this leads to process instabilities due to microbial populations shifting between NH₃-tolerant and NH₃-sensitive micro-organisms.

In addition, acetoclastic methanogens population shifting towards NH₃-tolerant micro-organisms is known to lead to VFA accumulation into the digester medium. Therefore, NH₃ content increase leads to VFA accumulation when the initially predominant microbial population comprises NH₃-sensitive micro-organisms such as acetoclastic methanogens.

VFA and total ammonia nitrogen were shown to contribute to foam formation - which negatively affects anaerobic treatments - by decreasing sludge surface tension and increasing foam stability (see Scientific Reports 7:13701, DOI:10.1038/s441598-017-14258-3, by He et al, October, 20 2017). More precisely, VFAs tend to rise and accumulate at the air-liquid interface with the biogas bubbles generated into the digester. This is the VFA accumulation that decreases the surface tension of digested liquid and promotes foam formation.

Instability may therefore not only translate into acidification of the anaerobic tank, but also into foaming issues.

To mitigate the NH₃ toxicity and potential foaming problems, conventional approaches are based on reducing the Volatile Solids (VS) content at the anaerobic tank inlet. This is for instance performed by diluting the incoming fresh sludge, *i.e.* downstream of a thermal pretreatment, or by maintaining the fresh sludge thickening to a value of DS (dry solid) content lower than 60 g/L.

If, despite these measures, the NH₃ content in the anaerobic tank is still above the toxicity threshold for NH₃-sensitive methanogens (about 80 mg/L and more), then process maintenance requires continuous selection pressure to be maintained to keep NH₃-sensitive methanogens as the predominant methanogen population, otherwise process instabilities may occur as explained above. But maintaining selection pressure usually involves adding an external source of ammonia such as urea, and/or by-passing the anaerobic tank to prevent dilution, at the expense of the process performance and/or of cost-efficiency.

An alternative approach to enhance CH₄ production has been proposed by Bajon Fernandez et al. (Environmental Technology, DOI:10.1080/0959330.2019.1597173), based on CO₂ injection into the digester. CO₂ injection is performed by bubbling a gas mainly containing CO₂ through the digester at the start of the digestion process. This solution seems promising in terms of CH₄ production, but does not provide an adapted solution to foaming problems. On the contrary, as indicated by He *et al*., such processes including further gas bubbling actually increase VFA accumulation at the air/liquid interface and lead to even more foam formation.

Consequently, there is still a need for improved digestion or fermentation processes, with increased stability, and with improved methane and/or VFA production/yields, involving in particular a fine control of the ratio between NH₃-tolerant and NH₃-sensitive microorganisms.

### SUMMARY OF THE INVENTION

The present invention thus aims to provide a solution to maintain NH₃ content in the digestion medium outside of its toxicity range.

The combined concentration of aqueous ammonia and ammonium can be measured on line, by colorimetry or by determination of ammonium in water by titration after steam distillation (NF-T90-015-1). This concentration is called total ammonia nitrogen (TAN).

The present invention also provides a solution to control the chemical equilibrium between aqueous ammonium NH₄⁺ and ammonia NH₃, thus ensuring methanogens performance while avoiding undesired side effects.

To this end, in a first aspect, the invention relates to a method for controlling NH3 content in an anaerobic medium of an anaerobic process comprising:
a) a step of providing organic material,
b) a step of subjecting said organic material of step a) to an anaerobic treatment, thereby producing a digestate and optionally biogas,
c) a step of solubilizing CO₂ from a CO₂-containing gas into a liquid medium comprising at least one microorganism population, thereby providing a CO₂-enriched liquid medium, and
d) a step of subjecting at least part of the CO₂-enriched liquid medium of step c) to the anaerobic treatment of step b).

As used herein, the "organic material" of step a) is usually a sludge, such as a sludge resulting from wastewater treatment, and/or organic waste. In a particular embodiment, the "organic material" of step a) is a sludge from a wastewater treatment plant.

The "microorganism population" of step c) are methanogens. Said at least one microorganism population of step c) typically comprises homoacetogenic bacteria, acetogenic methanogens, hydrogenotrophic methanogens, carboxydotrophic acetogens, acetogenic methanogens, or mixtures thereof.

As used herein, an "anaerobic treatment" is understood as anaerobic digestion, or fermentation, which may be considered as a partial anaerobic digestion. The anaerobic treatment of the present invention is typically carried out in an anaerobic tank, containing the anaerobic medium wherein the anaerobic treatment takes place. The anaerobic medium comprises a liquid phase and a solid phase, said solid phase comprising microorganisms, in particular responsible for treating the organic matter and producing the biogas.

"Fermentation" is a process well-known in the art and may be defined as a biological anaerobic process extracting energy from carbohydrates in the absence of oxygen, to produce small molecules (organic substrates), in particular RBCs, through the action of enzymes in particular. No CH₄ is produced, or only traces amounts. There are five main types of fermentation:
a. Alcoholic Fermentation, yielding mainly ethanol,
b. Lactic Acid Fermentation, yielding lactate,
c. Propionic Acid Fermentation, yielding propionate,
d. Butyric Acid / Butanol Fermentation, yielding butyrate and butanol,
e. Mixed Acid Fermentation, yielding VFAs (mainly acetate, but also propionate, lactate, butyrate).

The fermentation process may be controlled by the retention time of the sludge into the anaerobic tank, temperature and pH in the anaerobic tank, as well as by the specific microbial population involved in the fermentation process (i.e. by the choice of microbial strains in the anaerobic tank).

Anaerobic digestion is a process involving microorganisms that break down biodegradable material in the absence of oxygen. This process produces a digestate and a gaseous fraction comprising methane, and typically consisting essentially of methane and CO₂, also called biogas.

Biogas is a gas resulting from the anaerobic digestion of organic matter through a process called methanisation. Biogas comprises CH₄ (typically 50-75%) and CO₂ (typically 50-25%). Synthesis gas, also called syngas, is a gas comprising CO, H₂, CO_{2,} and generally a small amount of CH₄, that usually results from the thermal degradation of biomass without combustion, through pyrolysis or gasification or hydrothermal gasification.

Anaerobic digestion produces biogas as a result of the biological fermentation of organic solids supplied with the feedstock. Digesters treating complex organic substrates achieve generally between 30 and 60% of solids reduction. The digestate may be dewatered to produce a cake with typically 20 to 30% solids.

Advantageously, the anaerobic digestion is a digestion of effluents containing soluble components only, in particular soluble carbon i.e. containing no more suspended solids. Advantageously but not necessarily, the suspended solids have been solubilized in a hydrothermal gasification (HTG) step. An optional dedicated anaerobic digestion may further take place, such as a UASB type (upflow anaerobic sludge blanket digestion), i.e. treating soluble carbon.

The anaerobic treatment is usually performed at pH conditions between 7,0 and 7,5, preferably between 7,0 and 7,2.

Methanogenic microorganisms are also called methanogens. Examples of methanogens are homoacetogenic bacteria, acetogenic methanogens, hydrogenotrophic methanogens, carboxydotrophic acetogens and acetogenic methanogens.

Conventionally, a "digestate" is the non-gaseous product of an anaerobic digestion, while the "fermentate" is the fermentation product. However, in the present invention, unless stated otherwise, the word "digestate" will encompass the non-gaseous product of the anaerobic treatment, that is, respectively a "conventional" digestate for a digestion, and a "fermentate" for a fermentation.

A digestate, and more specifically a fermentate, comprises RBCs, and more particularly VFAs or other fermentation products such as lower alcohols - in particular of formula R-OH with R representing a saturated, linear or ramified C₁-C₄ hydrocarbon chain, notably ethanol or butanol - depending on the selected fermentation pathway.

"RBCs" or "readily biodegradable carbons" are well known from the person of skill in the art. They are for instance defined in "Activated Sludge Models ASM1, ASM2 and ASM3", edited by the IWA task group on mathematical modelling for design and operation of biological wastewater treatment, Henze et al (2000), ISBN 1 900222 24 8. Examples of readily biodegradable carbons are volatile fatty acids. RBCs may be generated by fermentation, for instance as in the unified fermentation and thickening (UFAT) process, disclosed in particular in US 6,387,264.

"VFAs" or "volatile fatty acids" are also well-known to the one of skill in the art. In particular, they include lower carboxylic acids, notably C₁-C₄ saturated, linear or ramified hydrocarbon chains substituted by a COOH group, such as lactic acid, butyric acid, propionic acid, and acetic acid.

The CO₂-containing gas may be syngas, biogas (in particular pretreated biogas such as CO₂-enriched biogas), or CO₂, for instance obtained from purification of syngas and/or biogas.

The method of the invention may further comprise a step of recirculating at least a part of the digestate of step b) into the liquid medium of step c).

In a variant where the anaerobic treatment of step b) is a digestion, the method of the invention may further comprise a step of separating CO₂ from the biogas produced during the anaerobic treatment step b), thereby providing a first CO₂ enriched gas, as well as a second CH₄-enriched gas, such as biomethane. Advantageously, said first CO₂ enriched gas is used as the CO₂-containing gas of step c). Alternatively, the CO₂-containing gas of step c) may comprise at least part of said first CO₂₋enriched gas.

Advantageously, the method of the invention further comprises a step of controlling a quantity of CO₂ to be solubilized in step c), as a function of a NH₃ concentration of the anaerobic medium or in the digestate of step b), or of at least part or of a sample of said liquid medium. Indeed, without wishing to be bound by theory, as equilibrium (1) depends from the pH, the pH value is in turn a good indicator of the NH3 concentration into the liquid medium. In a particular embodiment, the NH₃ concentration is calculated from an inline TAN measurement in the digestate, preferably measured in the at least one part of the digestate recirculated into the liquid medium of the liquid bath. In another particular embodiment, the NH₃ concentration is calculated from an inline pH measurement in the anaerobic medium or in the digestate of step b), preferably measured in the at least one part of the digestate recirculated into the liquid medium of the liquid bath.

The "NH3 toxicity level" is usually understood as follows. In case the predominant micro-organisms population in the anaerobic medium comprises NH3-sensitive micro-organisms: above about 80 mg/L (*i.e.* 80 mg of NH3 per liter). In this case, the NH₃ level in the anaerobic tank is preferably maintained to a concentration of 80 mg/L or less.

In case the predominant micro-organisms population in the anaerobic medium comprises NH3-resistant micro-organisms: above about 750 mg/L, preferably 600, and more preferably 500 mg/L. In this case, the NH₃ level in the anaerobic tank is preferably maintained to a concentration of 100 mg/L or more, and usually of 300 mg/L or more, and it is preferably of 750 mg/L or less, more preferably of 600 mg/L or less, and even more preferably of 500 mg/L or less.

Preferably, the solubilizing step c) comprises a step c1) of injecting a CO₂-containing gas into a membrane reactor configured to be placed inside a liquid bath comprising said liquid medium, said liquid medium comprising said at least one microorganism population, said membrane reactor comprising at least one hollow fiber arranged in such a way that, when the membrane reactor is in contact with the liquid medium, a biofilm is formed by said at least one microorganism population around the at least one hollow fiber.

Advantageously, in this embodiment, the method of the invention further comprises a step of controlling a quantity of CO₂ injected into the membrane reactor of step c), as a function of a NH₃ concentration in the at least a part of the digestate recirculated into the liquid medium of the liquid bath.

In an embodiment, the method of the invention may comprise a step of subjecting organic material of step a) to a thermal treatment prior to step b), thereby generating at least:
- syngas, and
- a liquid effluent, said liquid effluent being then subjected to the anaerobic treatment of step b).

Thermal treatments are known in the art. Examples of "thermal treatments" are THP (Thermal Hydrolysis Process), HTC (Hydrothermal Carbonization), HTL (Hydrothermal Liquefaction), HTG (Hydrothermal Gasification), pyrolysis, gasification.

Preferably, said thermal treatment is hydrothermal gasification (also called HTG).

In this embodiment, said syngas may be used as the CO2-containing gas of step c). Alternatively, the CO2-containing gas of step c) may comprise at least part of said syngas.

In an embodiment wherein the thermal treatment is a hydrothermal gasification, the method of the invention may further comprise a step of producing an inorganic solid residue and a filtrate F1, optionally containing biodegradable carbons such as VFAs, a step of subjecting the filtrate F1 to the fermentation or the anaerobic digestion.

Hydrothermal gasification (HTG) is a thermal depolymerization process used to convert carbonaceous material - in particular wet biomass - into a mixture comprising only small molecules under high to moderate temperature and high pressure. It is well known in the art, and is for instance described in WO2013/030026 or WO2013/030027. The HTG step is usually carried out in an HTG reactor. It is also described in WO99/00334 or and US2017/0342327 in combination with an oxidation step.

During HTG, carbon and hydrogen of a carbonaceous material, such as biomass, are thermo-chemically converted into hydrophobic compounds with low viscosity and high solubility. In a first embodiment, a limited amount of oxygen or air is introduced into the reactor to allow some of the organic material to be "burned" to produce carbon dioxide and energy, which drives a second reaction that converts further organic material to hydrogen and additional carbon dioxide. Alternatively, energy generation can also be provided externally without having to inject oxygen or air in the system. Further reactions occur when the formed carbon monoxide and residual water from the organic material react to form methane and excess carbon dioxide. This third reaction occurs more abundantly in reactors that increase the residence time of the reactive gases and organic materials, as well as heat and pressure.

As a result, HTG may be regarded as 1) a phase separation, separating inorganic solid residues (namely ashes and salts) from the supercritical phase, which upon cooling yields a gaseous phase and a liquid phase, and 2) a depolymerisation process transforming organic carbonaceous material to more easily biodegradable material, such as RBCs.

Processing conditions (in particular temperature, pressure, product concentration and to a less extent residence time) of the HTG may be adjusted to not only produce inorganic solid residues (ashes) and a gaseous fraction containing CH₄, CO, CO₂ and H₂ (syngas), but to also produce a filtrate (also referred to as "biocrude") containing mostly RBCs, particularly VFAs.

The invention further relates to an installation for NH3 content control in an anaerobic tank comprising:
- an anaerobic tank, suitable for anaerobic treatment, having a first inlet, a second inlet, a first outlet and a second outlet, the anaerobic tank being configured to be fed at the first inlet with organic material, and
   to produce a digestate recovered at the first outlet and optionally biogas recovered at the second outlet,
- a membrane reactor configured to be placed inside a liquid bath comprising a liquid medium, the liquid medium comprising at least one microorganism population,
   said membrane reactor comprising at least one hollow fiber arranged in such a way that, when the membrane reactor is in contact with the liquid medium, a biofilm is formed around the at least one hollow fiber,
   said membrane reactor further having a first inlet, a first outlet and a lateral outlet,
- and further comprising a CO₂ source in fluid connection with the first inlet of the membrane reactor,
   the membrane reactor being configured so that the CO₂ source fed at the first inlet of the membrane reactor flows into the at least one hollow fiber, so as to solubilize CO₂ into the liquid medium,
- wherein the second inlet of the anaerobic tank is in fluid connection with the lateral outlet of the membrane reactor, and
   the anaerobic tank is configured to be fed at the second inlet with at least part of the liquid medium comprising solubilized CO₂ recovered at the lateral outlet of the membrane reactor.

The CO2 source is to be understood as any CO2 supply. The CO2 source may be isolated CO2. It may also be a CO2-containing gas, whatever its CO2 content is. The isolated CO2 or the CO2-containing gas may be either provided as a by-product of the method of the invention, or as an external source.

Preferably, the installation of the invention comprises a recirculation loop configured to recirculate at least a part of the digestate into the liquid medium of the liquid bath.

In an embodiment, the installation of the invention comprises a unit for thermal treatment having a first inlet and a first outlet and a second outlet, and configured to be fed at the first inlet with organic material, the unit for thermal treatment being configured for generating at least:
- a thermally treated organic material at the first outlet, the first inlet of the anaerobic tank being in fluid connection with the first outlet of the unit for thermal treatment;
- syngas recovered at the second outlet, the first inlet of the membrane reactor (16) being optionally in fluid connection with the second outlet of the unit for thermal treatment, and the CO₂ source comprising syngas.
In this embodiment, the anaerobic tank is fed at least in part with thermally treated organic material.

In a variant, the installation of the invention comprises a gas separator having a first inlet and a first outlet, the first inlet being in fluid connection with the second outlet of the anaerobic tank, the gas separator being configured to separate CO₂ from the biogas recovered at the second outlet of the anaerobic tank, the first outlet of the gas separator being in fluid connection with the first inlet of the membrane reactor so as to feed the membrane reactor at the first inlet with the CO₂ separated from the biogas. The gas separator is preferably a membrane gas separator.

Preferably, the installation of the invention comprises a control loop to control a quantity of CO₂ fed at the first inlet of the membrane reactor as a function of a NH3 concentration in the anaerobic tank.

Preferably, the installation of the invention comprises a control loop to control a quantity of CO₂ fed at the first inlet of the membrane reactor as a function of a NH3 concentration in the recirculation loop.

In an embodiment wherein the unit for thermal treatment is a HTG reactor suitable for hydrothermal gasification, further having a second and third outlets, the HTG reactor is configured to further produce an inorganic solid residue, recovered at the second outlet, a filtrate F1, optionally containing readily biodegradable carbons such as VFAs, recovered at the third outlet, the third outlet being in fluid connection with the first inlet of the anaerobic tank, the anaerobic tank being configured to be fed at the first inlet with filtrate F1.

According to the invention, the at least one microorganism population in the liquid bath typically comprises homoacetogenic bacteria, acetogenic methanogens, hydrogenotrophic methanogens, carboxydotrophic acetogens, acetogenic methanogens, or mixtures thereof.

### BRIEF DESCRIPTION OF THE DRAWINGS

The accompanying drawings illustrate various non-limiting, exemplary, innovative aspects in accordance with the present description:
- Figure 1 schematically represents a block diagram with the step(s) of a method for controlling NH3 content in an anaerobic medium according to the invention;
- Figure 2 schematically represents a block diagram with the optional steps of a method for controlling NH3 content in an anaerobic medium according to the invention;
- Figures 3A and 3B schematically represent two embodiments of the installation for NH3 content control in an anaerobic tank according to the invention;
- Figures 4A and 4B schematically represent an embodiment of the membrane reactor of the installation for NH3 content control in an anaerobic tank according to the invention;
- Figure 5 schematically represents another embodiment of the installation for NH3 content control in an anaerobic tank according to the invention;
- Figure 6 schematically represents another embodiment of the installation for NH3 content control in an anaerobic tank according to the invention;
- Figure 7 schematically represents another embodiment of the installation for NH3 content control in an anaerobic tank according to the invention;
- Figure 8 schematically represents another embodiment of the installation for NH3 content control in an anaerobic tank according to the invention;
- Figure 9 schematically represents another embodiment of the installation for NH3 content control in an anaerobic tank according to the invention.

For the sake of clarity, the same elements have the same references in the various figures.

### DETAILED DESCRIPTION

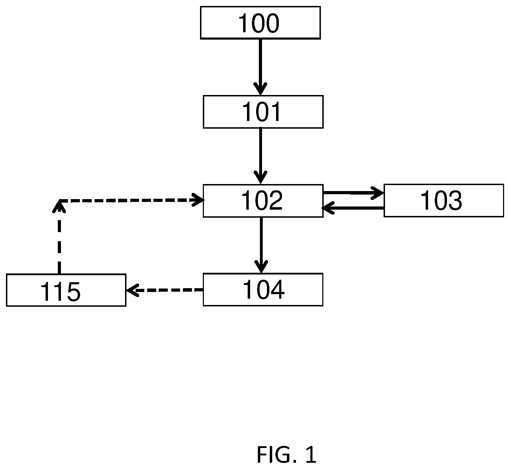

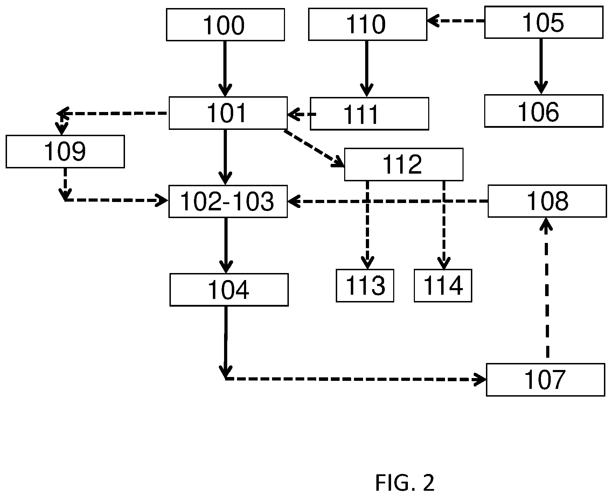

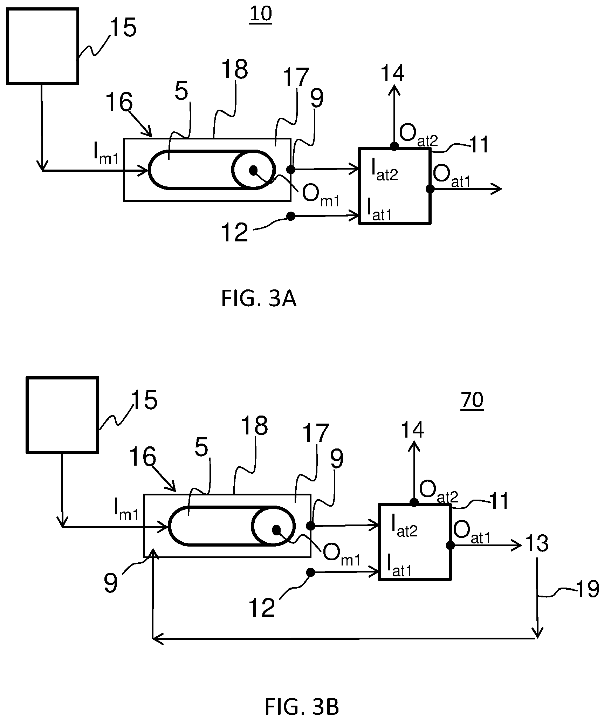

Figure 1 schematically represents a block diagram with the step(s) of a method for controlling NH3 content in an anaerobic medium according to the invention. The steps of the method of the invention are described with Figures 1 and 2 and more details with the involved processes are given below in relation to the description of figures 3A to 9.

The method for controlling NH3 content in the anaerobic tank 11 comprises a step 100 of providing organic material 12, a step 101 of subjecting the organic material 12 of step 100 to an anaerobic treatment, for example a fermentation or an anaerobic digestion, thereby producing a digestate 13 and optionally biogas 14. It is reminded that biogas comprises methane and CO2. It further comprises a step 102 of solubilizing CO2 from a CO2-containing gas into a liquid medium 17 comprising at least one microorganism population, thereby providing a CO2-enriched liquid medium. The method of the invention comprises a step 104 of subjecting at least part of the CO2-enriched liquid medium to the anaerobic treatment, for example the fermentation or the anaerobic digestion. The method according to the invention enables to control the NH3 content in the anaerobic digester by injection of carbon dioxide, without foam formation thanks to the solubilized carbon dioxide.

The solubilizing step 102 comprises a step 103 of injecting a CO2-containing gas into a membrane reactor 16 configured to be placed inside a liquid bath 18 comprising a liquid medium 17, the liquid media 17 comprising at least one microorganism population, the membrane reactor 16 comprising at least one hollow fiber 5 arranged in such a way that, when the membrane reactor 16 is in contact with the liquid medium 17, a biofilm is formed around the at least one hollow fiber 5.

According to another embodiment of the invention, the method for controlling NH3 content in an anaerobic medium further comprises a step 115 of recirculating at least a part of the digestate 13 into the liquid medium 17 of the liquid bath 18. Recirculating at least a part of the digestate 13 after the digestion/fermentation into the liquid medium 17 that is at least partly introduced into the anaerobic tank 11 avoids the dilution of the anaerobic medium inside the anaerobic tank 11, thus ensuring an Hydraulic Retention Time (HRT) long enough in the anaerobic tank 11.

Figure 2 schematically represents a block diagram with the optional steps of a method for controlling NH3 content in an anaerobic medium according to the invention. The method for controlling NH3 content in an anaerobic medium may further comprise a step 105 of subjecting organic material 22 to a thermal treatment thereby generating at least syngas 23, the CO2-containing gas comprising said syngas 23. CO2 injected into the membrane reactor 16 during the step 103 may therefore be provided by syngas 23.

The method according to the invention may further comprise a step 107 of separating CO2 from the biogas 14 produced during the anaerobic treatment step 101, for example fermentation or anaerobic digestion, thereby providing a first CO2 enriched gas and a second CH4-enriched gas, such as biomethane, and a step 108 of providing at least part of said first CO2 enriched gas of step 107 as the CO2-containing gas at the step 107. The step 108 corresponds to the feeding of the membrane reactor 16 with said first CO2 enriched gas of step 107. CO2 injected into the membrane reactor 16 during the step 102 may therefore be provided by at least part of the carbon dioxide of the biogas 14.

In another embodiment of the invention, the method comprises a step 109 of controlling a quantity of CO2 injected during the step 102 of injecting CO2 into the membrane reactor 16 as a function of a NH3 concentration during the fermentation or the anaerobic digestion. This step enables to adapt the quantity of CO2 injected depending on the necessity or not to render the anaerobic medium more or less acid, thereby influencing the NH3 concentration.

Advantageously, the method according to the invention comprises a step 109 of controlling a quantity of CO2 injected during the step 102 of injecting CO2 into the membrane reactor 16 as a function of a NH3 concentration in the at least a part of the digestate 13 recirculated into the liquid medium 17 of the liquid bath 18. This step enables to adapt the quantity of CO2 injected depending on the necessity or not to render the anaerobic medium more or less acid, thereby influencing the NH3 concentration.

In another embodiment wherein the thermal treatment is a hydrothermal gasification, the method further comprises a step 110 of producing an inorganic solid residue 12 and a filtrate F1, optionally containing biodegradable carbons such as VFAs, a step 111 of subjecting the filtrate F1 to the fermentation or the anaerobic digestion. The anaerobic treatment minimizes the HTG energy requirements by reducing the need to fully convert all organic matter into syngas, and is improved by the presence of RBCs into the filtrate F1.

The at least one microorganism population in the liquid bath of step c) preferably comprises homoacetogenic bacteria, acetogenic methanogens, hydrogenotrophic methanogens, carboxydotrophic acetogens, acetogenic methanogens, or mixtures thereof.

Figures 3A and 3B schematically represent two embodiments of the installation for NH3 content control in an anaerobic tank according to the invention. Figure 3A represents an installation 10 for NH3 content control in an anaerobic tank 11 comprising an anaerobic tank 11, suitable for fermentation or anaerobic digestion, having a first inlet lat1, a second inlet lat2, a first outlet Oat1 and a second outlet Oat2, the anaerobic tank 11 being configured to be fed at the first inlet lat1 with organic material 12 and to produce a digestate 13 recovered at the first outlet Oat1 and optionally biogas 14 comprising methane and CO2 (carbon dioxide) recovered at the second outlet Oat2. The installation 10 comprises a membrane reactor 16 configured to be placed inside a liquid bath 18 comprising a liquid medium 17, the liquid medium 17 comprising at least one microorganism population, the membrane reactor 16 comprising at least one hollow fiber 5 arranged in such a way that, when the membrane reactor 16 is in contact with the liquid medium 17, a biofilm is formed around the at least one hollow fiber, the membrane reactor 16 having a first inlet Im1 and a first outlet Om1, and also lateral inlet/outlet 9 towards/from the liquid medium 17. According to the invention, the installation 10 comprises a CO2 source 15 in fluid connection with the first inlet Im1 of the membrane reactor 16, the membrane reactor 16 being configured so that the CO2 source fed at the first inlet Im1 of the membrane reactor 16 flows into the at least one hollow fiber, so as to solubilize CO2 into the liquid medium 17. The second inlet lat2 of the anaerobic tank 11 is in fluid connection with the lateral outlet 9 of the membrane reactor 16 and the anaerobic tank 11 is configured to be fed at the second inlet lat2 with at least part of the liquid medium 17 comprising solubilized CO2 recovered at the lateral outlet 9 of the membrane reactor 16.

The CO2 source 15 may be an external source of pure CO2 or an external source of a gas mixture comprising CO2. Instead of an external source of CO2, or additionally to the external source CO2, the CO2 source 15 may comprise an internal source of CO2. The CO2 may be a component of syngas or biogas produced within the installation. In this case, the gas may be treated or separated to extract CO2 as a CO2 source 15 to be fed to the membrane reactor 16.

The installation according to the invention enables an indirect CO2 diffusion via a hollow fiber membrane reactor into the liquid medium of the liquid bath. CO2 is diffused through the membrane and is solubilized in the liquid medium instead of being sparged. In other words, the installation of the invention enables the diffusion of the carbon dioxide through the membrane, thus leading to the solubilisation of the carbon dioxide without bubbling in the liquid medium of the liquid bath of the membrane reactor. At least part of the liquid medium comprising solubilized CO2 is introduced into the anaerobic tank. This results in the enhancement of the anaerobic digestion process through carbon dioxide enrichment, without undesired effects such as foaming constated further to carbon dioxide injection under the form of bubbles.

Figure 3B represents an installation 70 for NH3 content control in an anaerobic tank 11. The installation 70 represented in Figure 3B comprises the same elements as the installation 10 represented in Figure 3A. Additionally, the installation 70 comprises a recirculation loop 19 configured to recirculate at least a part of the digestate 13 into the liquid medium 17 of the liquid bath 18. The recirculation loop 19 may also be called a digester recirculation line. The installation 70 may advantageously comprise a heating or cooling heat exchanger on the recirculation loop 19. Recirculating at least a part of the digestate 13 after the digestion/fermentation into the liquid medium 17 that is at least partly introduced into the anaerobic tank 11 avoids the dilution of the anaerobic medium inside the anaerobic tank 11, thus ensuring an Hydraulic Retention Time (HRT) long enough in the anaerobic tank 11.

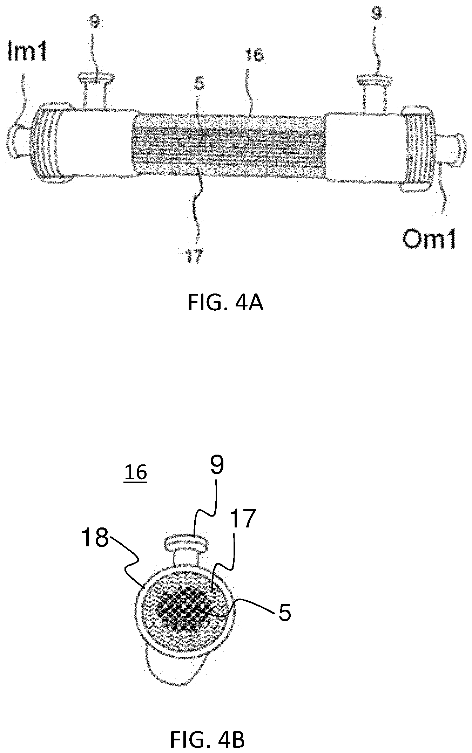

Figures 4A and 4B schematically represent an embodiment of the membrane reactor 16 of the installation for NH3 content control in an anaerobic tank according to the invention.

Figure 4B represents a side view of the membrane reactor 16. The reactor 16 comprises two lateral inlet/outlet 9 for fluid feeding/recovering the liquid medium 17. The inlet stream enters the membrane reactor 16 by the inlet port 6 and flows into the hollow fibers 5. Each hollow fiber has a nominal pore size, an inner and outer diameter and a useful length, as well as an outer surface area. Figure 4A represents a cut section of the membrane reactor 16. The feeding gas (i.e. carbon dioxide or syngas) is supplied at one end of the cartridge and conveyed inside the fibers 5 to diffuse through the membrane, without gas recirculation. The lateral inlet 9 is fed with the liquid medium 17 as inlet of the liquid, which is a defined media solution for a predetermined average hydraulic retention time. Liquid can flow against or parallel to the gas flow. The pH of the liquid medium 17 as well as the liquid pressure may be monitored and controlled by using corresponding devices. The fiber membrane serves as a support for the microbial population as well as an interface between the gas and the liquid phases.

The liquid medium 17 can be water, either treated water or partially treated water, which contains various populations of microorganisms. The liquid medium 17 may comprise suspended particulates. When the membrane reactor 16 is placed on the recirculation loop 19 of at least a part of the digestate 13, depending on the type of digestate 13, the liquid medium 17 may comprise between 1 and 10% of solid matter, typically between 2 and 8%. When operating, a biofilm develops on the membrane made of fibers, depending on the inlet stream in the membrane reactor. In other words, only population of microorganisms in adequacy with the substrate in the reactor can grow. The culture of microorganisms fixes on the membrane. Feeding CO2 at the inlet of the membrane reactor 16 enables the solubilization of CO2 in the liquid medium 17 thanks to the membrane technology. The CO2 diffuses through the biofilm enveloping the hollow fibers and is solubilized into the liquid medium 17 of the membrane reactor 16. This means that CO2 dissolves in the liquid medium 17 to form a homogeneous mixture of liquid medium 17 containing solubilized CO2.

Multiple reactions can occur via two paths, either direct methanogenesis driven by hydrogenogenic methanogens: 4H2+CO2 → CH4 + H2O). H2 is generated by fermentation and by the chemical equilibrium CO2+H20 <-> H2CO3 <-> HCO3 + H+. Or, by homoacetogenic bacteria, which are strictly anaerobic microorganisms that catalyze the formation of acetate from C1 units (typically CO2). This is carried out via the acetyl-CoA pathway. Once acetate is produced, it is consumed by acetoclastic methanogens (conventional methanogensis).

These reactions take into account the conversion of the gas components when a gas such as syngas is fed at the inlet of the membrane reactor 16, pointing out that methane may be recovered at a gas outlet of the membrane reactor. This is one possible role played by the membrane reactor. Nevertheless, the principle of the invention is based on another role of the membrane reactor which consists in injecting CO2 at the inlet of the membrane reactor 16 to obtain a liquid medium 17 saturated in solubilized CO2. This liquid medium 17 saturated in solubilized CO2 is fed at an inlet of the anaerobic tank 11. The introduction of at least part of the liquid medium 17 saturated in CO2 enables to control the pH inside the anaerobic tank 11, i.e. the pH of the digester medium contained in the anaerobic tank. The control of the pH of the digester medium enables to drive the concentrations of ammonia and ammonium. In other words, the injection of solubilized CO2 into the digester medium reduces to NH3 concentration in favor of NH4+. The fact that the CO2 is solubilized presents the advantage, in comparison to traditional CO2 sparging, of avoiding foam formation inside the anaerobic tank.

Thanks to the biofilm formed in the membrane reactor 16, the carbon dioxide contained in the inlet stream, i.e. the CO2 fed at the first inlet of the membrane reactor 16, is converted into CH₄ and solubilized CO2 in the liquid medium 17. This liquid medium 17 containing solubilized CO2 is extracted from the liquid bath 18 and fed to the anaerobic tank 11.

It can be noted that the disposal of the hollow fibers 5 inside the membrane reactor 16 may be different. The idea is to have an increasing number of modules containing the fibers as a function of the flow of syngas to be processed. The disposal of the hollow fibers should enable flexibility in their implementation and operation for maintenance, without causing too much pressure drop. As another example, the membrane reactor may comprise a central longitudinal fiber receiving the input syngas and feeding, at regular spatial intervals or not, series of fibers extending perpendicularly to the central fiber. The advantage is then that it is possible to maintain a certain degree of conversion of the syngas to methane even if one is limited in length of reactor, precisely by playing on the exchange surface perpendicular to the central axis.

As the liquid medium 17 should contain suitable bacteria, it can be fed by an annex culture medium of bacteria.

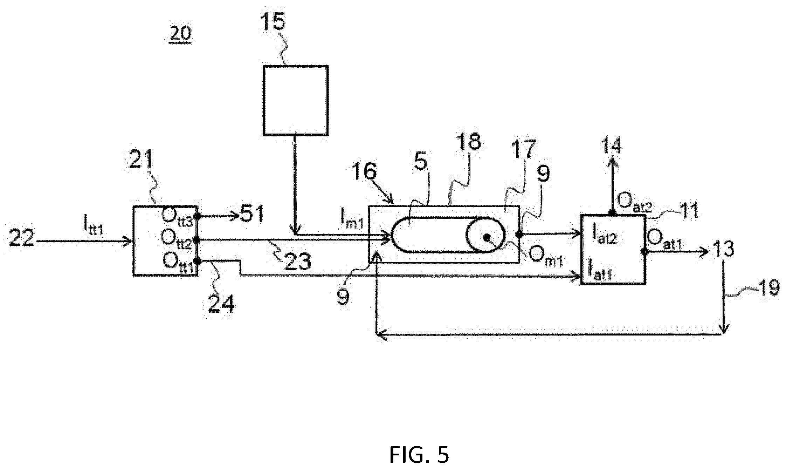

Figure 5 schematically represents another embodiment of the installation 20 for NH3 content control in an anaerobic tank 11 according to the invention. The installation 20 represented in Figure 5 comprises the same elements as the installation 70 represented in Figure 3B. As mentioned previously, the recirculation loop 19 of the digestate 13 into the liquid medium 17 is an optional, although preferred, element of the installation. In the following, the embodiments of the invention are presented with the recirculation loop 19, but the invention also concerns the same embodiments without the recirculation loop 19.

The installation 20 further comprises a unit 21 for thermal treatment having a first inlet Itt1 and a first outlet Ott1 and a second outlet Ott2 and configured to be fed at the first inlet Itt1 with organic material 22, the unit 21 for thermal treatment being configured for generating at least:
- a thermally treated organic material 24 at the first outlet Ott1, the first inlet lat1 of the anaerobic tank 11 being in fluid connection with the first outlet Ott1 of the unit for thermal treatment 21, and
- syngas 23 recovered at the second outlet Ott2.

The first inlet Im1 of the membrane reactor 16 is optionally in fluid connection with the second outlet Ott2 of the unit 21 for thermal treatment, and the CO2 source 15 comprises syngas 23.

The unit 21 for thermal treatment is any unit capable of a thermal treatment or conditioning such as THP (Thermal Hydrolysis Process), HTC (Hydrothermal Carbonization), HTL (Hydrothermal Liquefaction), HTG (Hydrothermal Gasification), pyrolysis, gasification.

The membrane reactor 16 is configured so that the syngas 23 generated at the unit 21 for thermal treatment flows at the first inlet Im1 of the membrane reactor 16 into the at least one hollow fiber 5, so as to convert the syngas 23 into methane recovered at the first outlet Om1 of the membrane reactor 16. In this embodiment, the membrane reactor 16 converts the inlet stream of syngas 23 into methane on the one hand and solubilizes at least a part of the CO2 contained in the inlet stream of syngas 23 into the liquid medium 17.

Depending on the type of unit 21 for thermal treatment, an inorganic solid residue, also called ashes, 51 may be recovered at a third outlet Ott3 of the unit 21 and a filtrate F1 may also be recovered at the first outlet Ott1 of the unit 21. This embodiment will be presented later on.

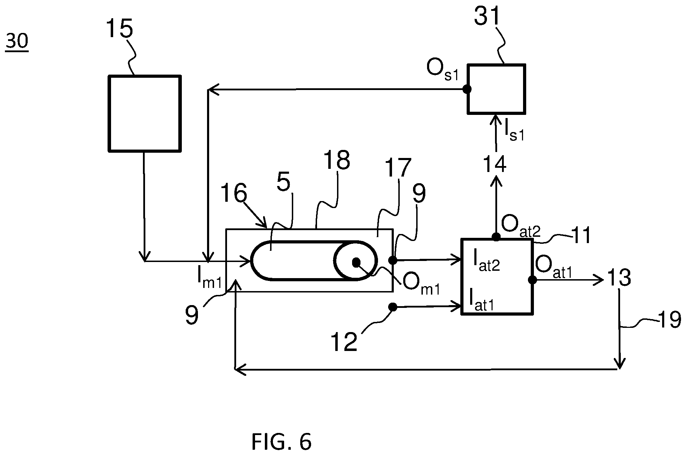

Figure 6 schematically represents another embodiment of the installation 30 for NH3 content control in an anaerobic tank 11 according to the invention. The installation 30 represented in Figure 6 comprises the same elements as the installation 70 represented in Figure 3B. The installation 30 comprises a gas separator 31 having a first inlet Is1 and a first outlet Os1, the first inlet Is1 being in fluid connection with the second outlet Oat2 of the anaerobic tank 11, the gas separator 31 being configured to separate CO2 from the biogas 14 recovered at the second outlet Oat2 of the anaerobic tank 11, the first outlet Os1 of the gas separator 31 being in fluid connection with the first inlet Im1 of the membrane reactor 16 so as to feed the membrane reactor 16 at the first inlet Im1 with the CO2 separated from the biogas 14. In this embodiment, biogas 14 recovered at an inlet of the anaerobic tank 11 is separated to recover the carbon dioxide of the biogas 14 and feed the membrane reactor 16 with the carbon dioxide of the biogas 14. The CO2 source 15 comprises at least part of the carbon dioxide of the biogas 14 and/or an external source of pure CO2 and/or another source of CO2, such as syngas 23 as explained before.

The installation 30 enables a recirculation of the CO2 recovered at the outlet of the anaerobic tank 11. Depending on the pH conditions in the anaerobic tank 11, this CO2 source may be the only source of CO2 of the installation. However, an additional CO2 source may be necessary to ensure the required quantity of solubilized CO2 in the liquid medium 17.

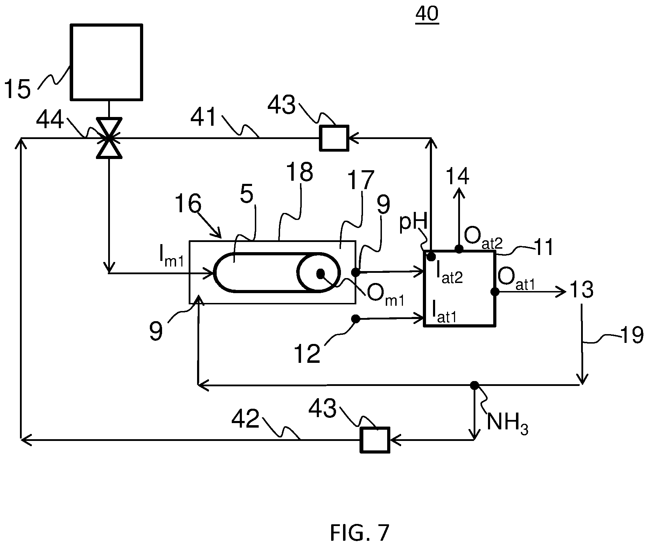

Figure 7 schematically represents another embodiment of the installation 40 for NH3 content control in an anaerobic tank 11 according to the invention. The installation 40 represented in Figure 7 comprises the same elements as the installation 70 represented in Figure 3B. The installation 40 further comprises a control loop 41 to control a quantity of CO2 fed at the first inlet Im1 of the membrane reactor 16 as a function of a NH3 concentration in the anaerobic tank 11. Alternatively, or additionally, the installation 40 may comprise a control loop 42 to control a quantity of CO2 fed at the first inlet Im1 of the membrane reactor 16 as a function of a NH3 concentration in the recirculation loop 19.

The aim of the invention is to control the ammonia (NH₃) content in the anaerobic tank 11. Indeed ammonia is toxic to the microorganisms that produce methane during the anaerobic treatment in the anaerobic tank. The relative concentration of NH₃ and NH₄⁺ in the anaerobic tank depends on the pH and temperature in the anaerobic tank, with higher pH values and temperatures favouring the formation of a molecular ammonia that is toxic to methanogens. In particular, a higher pH value leads to higher ammonia concentrations, thus increasing toxicity for the microorganisms.

The toxicity level depends on the microorganisms population present in the anaerobic tank. As mentioned before, it is about 80 mg/L (i.e. 80 mg of NH3 per liter) for acetoclastic methanogens (Methanosaetaceae), and about 750 mg/L (preferably 600, and more preferably 500 mg/L NH3) for hydrogen-utilizing methanogens (Methanosarcinaceae, Methanomicrobiales, Methanobacteriales), the latter ones showing a higher tolerance to NH3.

The formation of ammonia leads in particular to reduced performances of the acetoclastic methanogens and to a microbial population shift towards a dominance of hydrogen-utilizing methanogens. In other words, the microorganisms population adapts depending on the conditions in the anaerobic tank. From this, it results that there are two operating ranges of NH3 concentration, the first one being a NH3 concentration lower than about 80 mg/L and the second one being greater than about 100 mg/L and lower than about 750 mg/L. These values are to be considered as an approximation. In any case, the range between about 80 and about 100 mg/L is to be avoided since the range of NH3 concentration leads to an unstable mode of anaerobic treatment.

The control loop 41 enables to control the quantity of CO2 fed at the first inlet Im1 of the membrane reactor 16 as a function of a NH3 concentration in the anaerobic tank 11. When the NH3 concentration is too high, or approaching the threshold (for example of 80 or 500 mg/L), increasing the quantity of CO2 fed in the membrane reactor 16 will increase the quantity of solubilized CO2 introduced in to the anaerobic tank 11. Since CO2 is a weak acid, the pH of the anaerobic medium in the anaerobic tank 11 will decrease, thereby increasing the NH4+ concentration and decreasing the NH3 concentration. In order to determine the NH3 concentration in the anaerobic tank, the installation comprises either a specific sensor configured to measure the NH3 concentration via the Redox potential, and/or a pH-meter that enables to determine the NH3 concentration via an abacus defined by a person of ordinary skill in the art. Another possibility to determine the NH3 concentration is the pH measurement together with the TAN (total ammonia nitrogen) thanks to sampling of anaerobic medium. This way to proceed enables to obtain a very precise concentration of NH3 in the anaerobic tank.

The control loop 42 enables to control the quantity of CO2 fed at the first inlet Im1 of the membrane reactor 16 as a function of a NH3 concentration in the recirculation loop 19. The NH3 concentration may be measured as explained before, that is to say through a NH3 sensor (or a pH-meter enabling to know the NH3 concentration based on internal abacuses previously defined by a person skilled in the art depending on the operating conditions of the installation).

The control loop 42 offers the advantage of being very representative of the NH3 concentration at the inlet of the membrane reactor in the liquid medium 17, that is further introduced into the anaerobic digester. Moreover the recirculation loop 19 is easily accessible to realize an in-line measurement of the NH3 concentration or a sampling.

The control loop(s) 41 and/or 42 enable(s) to drive the CO2 quantity injected into the membrane reactor 16, that is further introduced into the anaerobic tank 11 under its solubilized form, to reduce or increase the NH3 concentration in the anaerobic tank thanks to the chemical processes explained before. Once the NH3 concentration is low enough (for example under 70 mg/L, or above 110 mg/L and lower than 500 mg/L) or high enough (for example greater than 800 mg/L), depending on the considered operating range, the calculator 43 on the control loop 41 and/or 42 actuates the valve 44 according to the quantity of CO2 to be injected into the membrane reactor 16. If the NH3 concentration in the anaerobic tank 11 is to be lowered, the valve 44 is actuated so as to inject more CO2 into the membrane reactor and if the NH3 concentration in the anaerobic tank 11 is to be increased, the valve 44 is actuated so as to inject less CO2 into the membrane reactor.

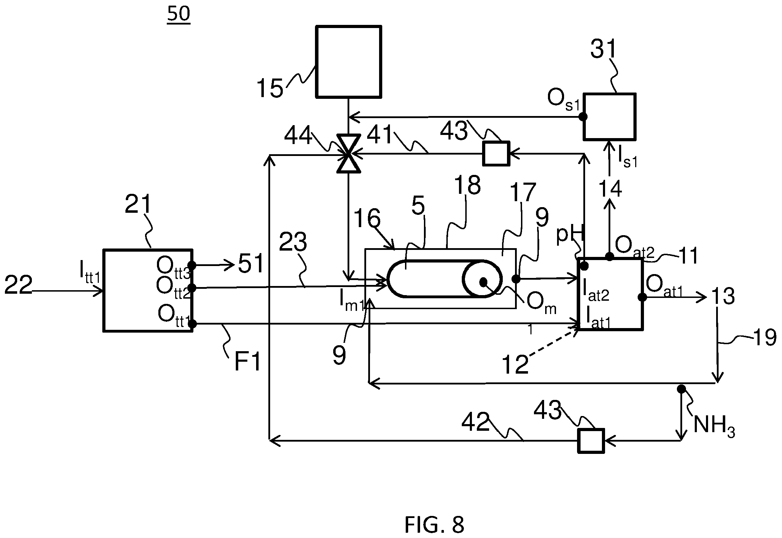

Figure 8 schematically represents another embodiment of the installation 50 for NH3 content control in an anaerobic tank 11 according to the invention. The installation 50 comprises the same elements as the installation 20. The unit 21 for thermal treatment is a HTG reactor suitable for hydrothermal gasification, further having a third outlet Oₜₜ₃. The HTG reactor 11 is configured to further produce an inorganic solid residue 51, recovered at the third outlet Ott3, and a filtrate F1, optionally containing readily biodegradable carbons such as VFAs, recovered at the first outlet Oₜₜ₁. The first outlet Ott1 is in fluid connection with the first inlet lat1 of the anaerobic tank 11, and the anaerobic tank 11 is configured to be fed at the first inlet lat1 with filtrate F1.

The installation 50 may comprise the control loop 41 to control the quantity of CO2 fed at the first inlet Im1 of the membrane reactor 16 as a function of a NH3 concentration in the anaerobic tank 11 and/or the control loop 42 to control the quantity of CO2 fed at the first inlet Im1 of the membrane reactor 16 as a function of the pH in the recirculation loop 19. In this embodiment, it is considered that all CO2 from syngas 23 is introduced into the membrane reactor and the NH3 concentration is controlled through the control loop 41 and/or 42 by actuation the valve 44 of the CO2 source 15. Nevertheless it is also possible to drive the quantity of CO2 from syngas 23 to be injected into the membrane reactor 16 with another control loop or by collecting all CO2 sources upstream the valve 44, as it is obvious for a person skilled in the art.

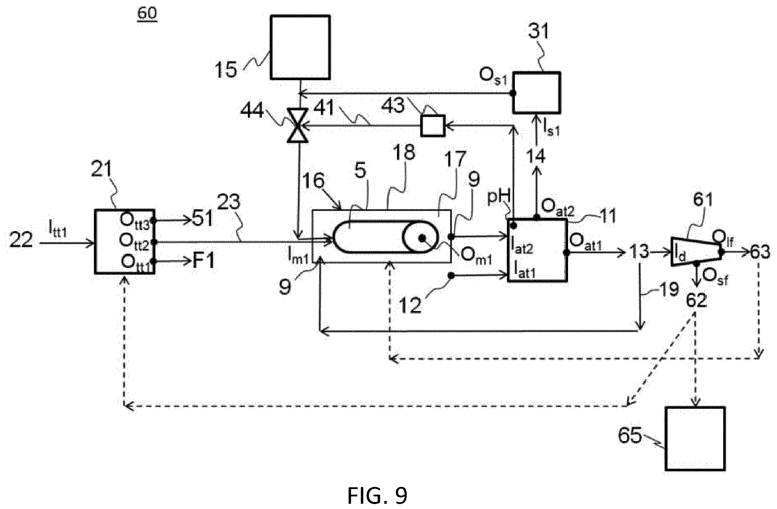

Figure 9 schematically represents another embodiment of the installation 60 for NH3 content control in an anaerobic tank 11 according to the invention. The installation 60 represented in Figure 9 comprises the same elements as the installation 50 represented in Figure 8. Nevertheless a person skilled in the art will understand that the installation 60 may also comprise only parts of the elements of the installation 50, for example only the elements of the installation 10, 20 or 70. The installation 60 comprises a dewatering unit 61 configured to dewater the digestate 13 from the anaerobic tank 11, so as to lead to a solid cake 62 and a liquid centrate 63. In this embodiment, either the digestate 13 is partly recirculated via the recirculation loop 19 or the digestate 13 is totally fed to the dewatering unit 61. The separation of at least part of the digestate 13 into the liquid centrate 63 and the solid cake 62 is of particular interest to separate suspended material in the digestate 13.

The installation 60 may be configured to inject at least a part of the liquid centrate 63 into the liquid medium 17 of the at least one membrane reactor 16. The liquid centrate 63 constitutes a culture medium to provide nutrient support and bacteria to the liquid medium 17 of the membrane reactor 16, so as to help the formation of the biofilm on the membrane.

The installation 60 may be configured to feed the solid cake 62 to the unit 21 for thermal treatment or to a second unit 65 for thermal treatment. The installation 60 enables an installation without any output since the product(s) of each treating element of the installation 60 is/are recirculated and treated.

The examples disclosed in this specification are only illustrative of some embodiments of the invention. They do not in any way limit the scope of said invention and all possible combinations of the presented embodiments are within the framework of the invention.

## Claims

1. A method for controlling NH3 content in an anaerobic medium of an anaerobic process (11), **characterized in that** it comprises:
• a step (100) of providing organic material (12),
• a step (101) of subjecting said organic material (12) to an anaerobic treatment, thereby producing a digestate (13) and optionally biogas (14),
• a step (102) of solubilizing CO₂ from a CO₂-containing gas into a liquid medium (17) comprising at least one microorganism population, thereby providing a CO₂-enriched liquid medium,
• a step (104) of subjecting at least part of the CO₂-enriched liquid medium to the anaerobic treatment.

2. The method of claim 1, wherein the solubilizing step (102) comprises:
• a step (103) of injecting a CO2-containing gas into a membrane reactor (16) configured to be placed inside a liquid bath (18) comprising a liquid medium (17), said liquid medium (17) comprising at least one microorganism population, said membrane reactor (16) comprising at least one hollow fiber (5) arranged in such a way that, when the membrane reactor (16) is in contact with the liquid medium (17), a biofilm is formed by said at least one microorganism population around the at least one hollow fiber (5).

3. The method of claim 1 or 2, further comprising a step (115) of recirculating at least a part of the digestate (13) into the liquid medium (17).

4. The method of any of claims 1 to 3, further comprising:
• a step (105) of subjecting organic material (22) to a thermal treatment thereby generating at least syngas (23), the CO₂-containing gas comprising said syngas (23).

5. The method of any one of claims 1 to 4, further comprising:
• a step (107) of separating CO₂ from the biogas (14) produced during the anaerobic treatment step (101), thereby providing a first CO₂ enriched gas and a second CH4-enriched gas, and
• a step (108) of providing said first CO₂ enriched gas of the step (107) of separating CO2 from the biogas as the CO2-containing gas at the step (102) of solubilizing CO2.

6. The method of any one of claims 2 to 5, further comprising a step (109) of controlling a quantity of CO2 injected during the step (103) of injecting CO2 into the membrane reactor (16) as a function of a NH3 concentration during the anaerobic treatment.

7. The method of any one of claims 2 to 6, further comprising a step (109) of controlling a quantity of CO2 injected during the step (102) of injecting CO2 into the membrane reactor (16) as a function of a NH3 concentration in the at least a part of the digestate (13) recirculated into the liquid medium (17) of the liquid bath (18).

8. The method of any one of claims 3 to 7, the thermal treatment being a hydrothermal gasification, the method further comprising:
• a step (110) of producing an inorganic solid residue (12) and a filtrate F1, optionally containing biodegradable carbons such as VFAs,
• a step (111) of subjecting the filtrate F1 to the anaerobic treatment.

9. Installation (10, 20, 30, 40, 50, 60, 70) for NH3 content control in an anaerobic tank (11) comprising:
• an anaerobic tank (11), suitable for fermentation or anaerobic digestion, having a first inlet (Iat1), a second inlet (Iat2), a first outlet (Oat1) and a second outlet (Oat2), the anaerobic tank (11) being configured to be fed at the first inlet (Iat1) with organic material (12) and to produce a digestate (13) recovered at the first outlet (Oat1) and biogas (14) comprising methane and CO2 recovered at the second outlet (Oat2),
• a membrane reactor (16) configured to be placed inside a liquid bath (18) comprising a liquid medium (17), the liquid medium (17) comprising at least one microorganism population, the membrane reactor (16) comprising at least one hollow fiber (5) arranged in such a way that, when the membrane reactor (16) is in contact with the liquid medium (17), a biofilm is formed around the at least one hollow fiber, the membrane reactor (16) having a first inlet (Im1), a first outlet (Om1) and a lateral outlet (9),
**characterized in that** the installation comprises a CO2 source (15) in fluid connection with the first inlet (Im1) of the membrane reactor (16), the membrane reactor (16) being configured so that the CO2 source fed at the first inlet (Im1) of the membrane reactor (16) flows into the at least one hollow fiber, so as to solubilize CO2 into the liquid medium (17),
and **in that** the second inlet (Iat2) of the anaerobic tank (11) is in fluid connection with the lateral outlet (9) of the membrane reactor (16) and the anaerobic tank (11) is configured to be fed at the second inlet (Iat2) with at least part of the liquid medium (17) comprising solubilized CO2 recovered at the lateral outlet (9) of the membrane reactor (16).

10. Installation (20, 30, 40, 50, 60, 70) according to claim 9, **characterized in that** it comprises a recirculation loop (19) configured to recirculate at least a part of the digestate (13) into the liquid medium (17) of the liquid bath (18).

11. Installation (20, 30, 40, 50, 60) according to claim 9 or 10, **characterized in that** it comprises a unit (21) for thermal treatment having a first inlet (Itt1) and a first outlet (Ott1) and a second outlet (Ott2) and configured to be fed at the first inlet (Itt1) with organic material (22), the unit (21) for thermal treatment being configured for generating at least:
• a thermally treated organic material (24) at the first outlet (Ott1), the first inlet (Iat1) of the anaerobic tank (11) being in fluid connection with the first outlet (Ott1) of the unit for thermal treatment (21); and
• syngas (23) recovered at the second outlet (Ott2),
**in that** the first inlet (Im1) of the membrane reactor (16) is optionally in fluid connection with the second outlet (Ott2) of the unit (21) for thermal treatment, and **in that** the CO2 source (15) comprises syngas (23).

12. Installation (30, 40, 50, 60) according to any one of claim 9 to 11, **characterized in that** it comprises a gas separator (31) having a first inlet (Is1) and a first outlet (Os1), the first inlet (Is1) being in fluid connection with the second outlet (Oat2) of the anaerobic tank (11), the gas separator (31) being configured to separate CO2 from the biogas (14) recovered at the second outlet (Oat2) of the anaerobic tank (11), the first outlet (Os1) of the gas separator (31) being in fluid connection with the first inlet (Im1) of the membrane reactor (16) so as to feed the membrane reactor (16) at the first inlet (Im1) with the CO2 separated from the biogas (14).

13. Installation (40, 50, 60), according to any one of claim 9 to 12, **characterized in that** it comprises a control loop (41) to control a quantity of CO2 fed at the first inlet (Im1) of the membrane reactor (16) as a function of a NH3 concentration in the anaerobic tank (11) or in the digestate (13).

14. Installation (40, 50, 60), according to any one of claim 10 to 13, **characterized in that** it comprises a control loop (42) to control a quantity of CO2 fed at the first inlet (Im1) of the membrane reactor (16) as a function of a NH3 concentration in the recirculation loop (19).

15. Installation (50, 60) according to any one of claim 11 to 14, the unit (21) for thermal treatment being a HTG reactor suitable for hydrothermal gasification, further having a third outlet (Oₜₜ₃), the HTG reactor (11) being configured to further produce:
• an inorganic solid residue (51), recovered at the third outlet (Ott3),
• a filtrate F1, optionally containing readily biodegradable carbons such as VFAs, recovered at the first outlet (Oₜₜ₁),
the anaerobic tank (11) being configured to be fed at the first inlet (Iat1) with filtrate F1.
